# EUROPÄISCHE PATENTSCHRIFT

(11) **EP 1 442 007 B1**
(45) Veröffentlichungstag und Bekanntmachung des Hinweises auf die Patenterteilung: **29.11.2006**
(21) Anmeldenummer: 02783005.8
(22) Anmeldetag: 25.10.2002
(51) Int. Cl.: C07C 209/10

(54) **VERFAHREN ZUR HERSTELLUNG VON ARYLAMINEN**
METHOD FOR THE PRODUCTION OF ARYLAMINES
PROCEDE DE PREPARATION D'ARYLAMINES

(30) Priorität: 30.10.2001 DE 10153450
(43) Veröffentlichungstag der Anmeldung: 04.08.2004
(73) Patentinhaber: Merck Patent GmbH, 64293 Darmstadt (DE)
(72) Erfinder: STÖSSEL, Philipp, 65929 Frankfurt (DE); SPREITZER, Hubert, 68519 Viernheim (DE); BECKER, Heinrich, 61479 Glashütten (DE)
(86) Internationale Anmeldenummer: PCT/EP2002/011942
(87) Internationale Veröffentlichungsnummer: WO 2003/037844

(56) Entgegenhaltungen:
- EP-A- 0 802 173
- WO-A-01/40147
- WO-A-99/12888
- DE-A- 3 521 770
- DE-A- 10 002 561
- PATENT ABSTRACTS OF JAPAN vol. 2000, no. 02, 29. Februar 2000 (2000-02-29) & JP 11 322679 A (TOSOH CORP), 24. November 1999 (1999-11-24)
- PATENT ABSTRACTS OF JAPAN vol. 2000, no. 04, 31. August 2000 (2000-08-31) & JP 2000 007689 A (KANKYO KAGAKU CENTER:KK), 11. Januar 2000 (2000-01-11)

## Beschreibung

Aromatische oder heteroaromatische Amine spielen eine bedeutende Rolle in der Industrie, so als Hilfsmittel bzw. in Vorprodukten für Pharmaka und Agrochemikalien, und in diversen Fein- und Elektronikchemikalien um nur einige Anwendungsfelder zu nennen. Vor allem auch in dem stark wachsenden Feld der organischen Halbleiter (z. B. Anwendungen in organischen bzw. polymeren Leuchtdioden, organischen Solarzellen, organischen ICs) sind gerade diese Verbindungen von herausragender Bedeutung.

Für die Herstellung sind verschiedene Alternativen bekannt, die jedoch nicht für alle Fälle eine ökonomisch und ökologisch befriedigende Lösung bieten. Die direkte Verknüpfung eines Amins mit einem Arylhalogenid, in einer sogenannten C-N-Kupplungsreaktion, wird häufig verwendet (vgl. Schema 1).

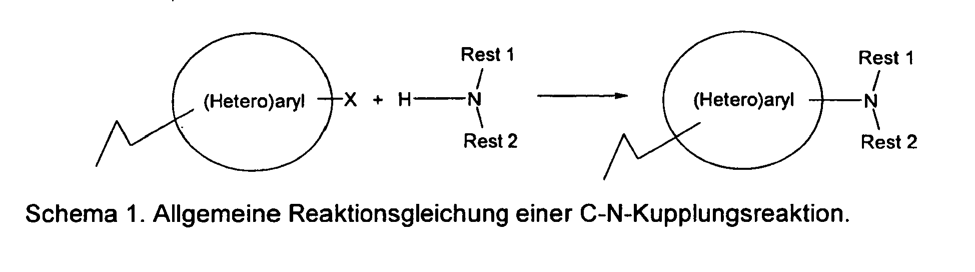

Obwohl diese Reaktion schon seit etlichen Jahren erforscht wird, mangelt es immer noch an einem industriell tauglichen Verfahren. Erste Ansätze in diese Richtung wurden von der Arbeitsgruppe um Buchwald et al., der Fa. Tosoh und der Fa. Du Pont de Nemours gemacht. Diese Arbeiten (vgl. US 5576460, EP-A- 0 802 173 und WO 01/40147) bilden den nächstliegenden Stand der Technik zur vorliegenden Anmeldung.

Buchwald et al. konnten in US 5576460 zeigen, daß durch die Umsetzung eines entsprechenden Halogenaromaten oder Halogenheteroaromaten mit einem entsprechenden primären bzw. sekundären Amin in Gegenwart einer Base, einer Palladiumverbindung, und gegebenenfalls eines tertiären Phosphins in einem Lösemittel sekundäre bzw. tertiäre Amine erhalten werden. So können unter diesen industrietauglichen Bedingungen Umsatzgrade von bis zu 90% erreicht werden.

In WO 99/12888 wird ein Verfahren zur Herstellung von Aryloligoaminen durch Umsetzung eines Amins mit einem aktivierten Aromaten in Gegenwart einer Palladiumkomponente und eines Phosphanliganden beschrieben. Dabei werden als Phosphanligand verschiedene Triarylphosphane verwendet. Die Ausbeuten liegen bei durchschnittlich 50 - 60 %.

In der Anmeldung WO 01/40147 konnte gezeigt werden, daß C-N-Kupplungen auch durch Phoshinoxide sekundärer Phosphine der allgemeinen Formel H(O)PR₂ katalysiert werden. Das Phosphinoxid kann als solches in den Prozeß eingebracht werden oder es wird vor der Zugabe zu den Reaktanden durch Hydrolyse mit Wasser aus entsprechenden hydrolyselabilen Phosphinoxid-Vorstufen dargestellt. Es werden für die Umsetzung von Chloraromaten mit primären Alkyl- und Arylaminen bzw. mit sekundären Alkylaminen Umsätze von bis zu 67 % beschrieben, wobei keine Angaben zur Kupplung von Halogenaromaten, insbesondere von Bromaromaten, mit Diarylaminen gemacht werden (vgl. auch Tabelle 1, Experimenteller Teil in WO 01/40147).

In EP-A-0 802 173 konnte gezeigt werden, daß die Kupplung sekundärer Amine mit entsprechenden Halogenaromaten oder Halogenheteroaromaten unter Verwendung von tertiären Phosphinen, insbesondere Trialkylphosphinen mit großem Kegelwinkel (Tolman-Winkel), zu noch besseren Ergebnissen führt. Setzt man als Trialkylphosphin mit großem Kegelwinkel z.B. Tri-*tert*-butylphosphin ein, werden technisch verwertbare Umsätze von bis zu 97% (vgl. insbesondere Beispiele 14, 20 -24, 34, 40, und 47 in EP-A-0 802 173) erreicht.

Für viele einfache Umsetzungen könnten die in EP-A-0 802 173 gefundenen und publizierten Bedingungen ausreichen, um ökonomisch sinnvolle Verfahren zu entwickeln. Problematisch ist jedoch die Darstellung multifunktioneller Verbindung. Multifunktionell im Sinne dieser Anmeldung soll bedeuten, daß eine Verbindung mehrere (z. B. zwei, drei, vier, fünf, usw.) gleiche oder gleichartige funktionelle Einheiten (Zentren) enthält, die in der entsprechenden Umsetzung alle in der gleichen Weise zu einem Produktmolekül reagieren. Mit der Umsetzung von multifunktionellen Verbindungen ist hier zunächst die Umsetzung einer multifunktionellen Verbindung mit mehreren monofunktionellen Verbindungen zu einer definierten "niedermolekularen" Verbindung gemeint (vgl. Schema 2).

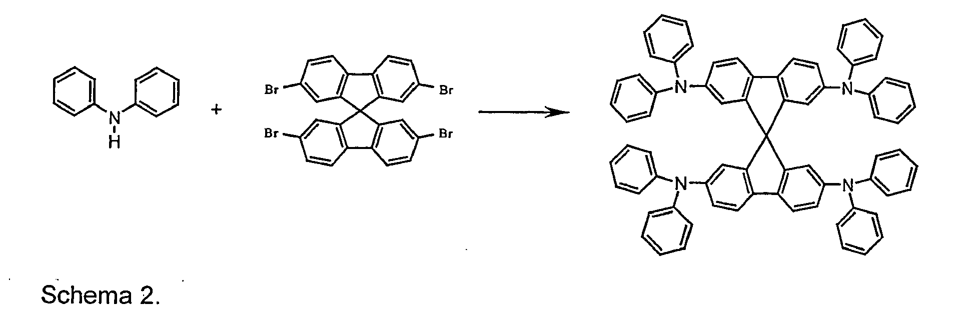

Beispiel für die Umsetzung einer multifunktionellen Verbindung (4 Zentren).

Bei der Darstellung multifunktioneller Verbindungen können selbst Umsatzgrade von bis zu 99% bei Regio-Selektivitäten von bis zu 99 % zu deutlichen Problemen führen.

Abweichungen von der vorgegebenen Regio-Chemie der Verknüpfung sind typische Nebenreaktionen der genannten Verfahren. Sie ergeben sich dadurch, daß die neu zu knüpfende C-N-Bindung nicht die Position einnimmt, die ehemals das Halogenatom des eingesetzten Halogenaromaten bzw. Halogenheteroaromaten einnahm, sondern daß die neu geküpfte C-N-Bindung um eine Position verschoben gebildet wird (vgl. Schema 3).

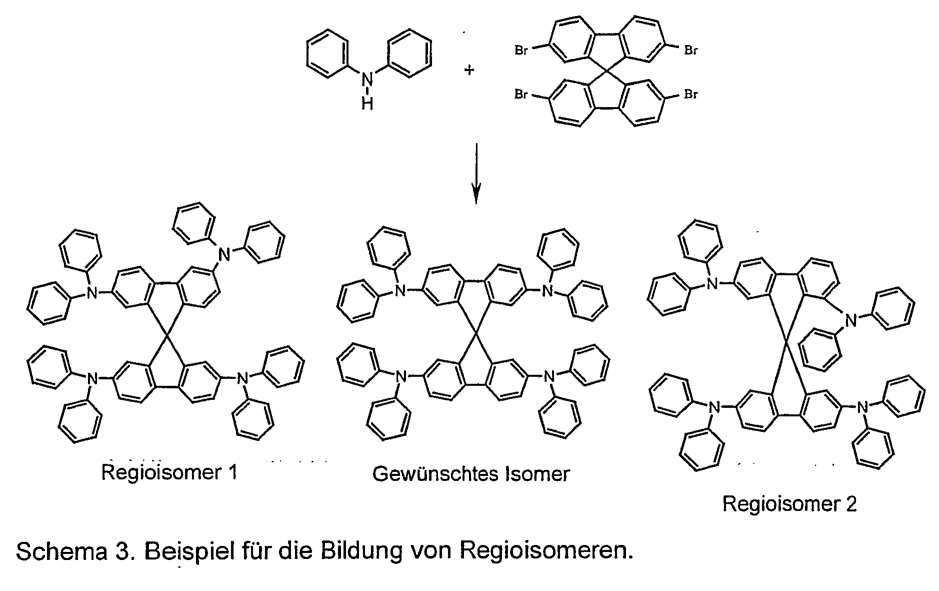

So führt beim Beispiel aus Schema 2 und 3 eine beispielhafte Umsatzrate (pro Einzelschritt) von 99% mit einer jeweiligen Regio-Selektivität von angenommenen 99% (d. h. pro Einzeladdition 0.99 x 0.99 = 98% Ausbeute an gewünschtem Substitutionsmuster) zu einer Gesamtausbeute von ca. 92%. Erfahrungsgemäß stellen die im obigen Beispiel genannten Umsatzgrade von bis zu 99% bei Regio-Selektivitäten von bis zu 99 % bei Verwendung der in US 5576460 und EP-A-0 802 173 beschriebenen Verfahren herausragende Einzelfälle dar, die in der Praxis nur bei ausgewählten Substraten, insbesondere bei solchen, die die Bildung von Regio-Isomeren durch sterische Gruppenhäufung unterdrücken (s. Beispiel 47 in EP-A-0 802 173), erreicht werden.

Die relativ hohe Ausbeute ist jedoch angesichts von Reinheitsanforderungen von +99.9% in z. B. elektronischen Anwendungen immer noch unbefriedigend, da die Aufreinigung bedingt durch die hohe Zahl verschiedener Nebenkomponenten, insbesondere der Regio-Isomere, die ein sehr ähnliches Kristallisationsverhalten wie das gewünschete Isomere zeigen, sehr aufwendig ist und sich stark ausbeutemindernd auswirkt (vgl. vorliegende Beschreibung Tabelle 1). Das oben dargelegte Beispiel zeigt klar auf, daß die Anwendung der genannten Verfahren für die Synthese hochreiner, multifunktioneller Verbindungen unzureichend ist.

Nachteilig an den nach EP-A-0 802 173 verwendeten tertiären Phosphinen in den Katalysatorsystemen ist ihre große Luftempfinglichkeit, die beim Tri-*tert-*butylphosphin so ausgeprägt ist, daß es bei Exposition dieses Phosphins an Luft zu einer spontanen Selbstentzündung kommen kann. Gerade bei technischen Anwendungen sind hier ganz besondere Vorkehrungen beim Umgang mit dieser Verbindung zu treffen, welche - neben dem ohnehin hohen Preis dieser Verbindung - einem Einsatz im größeren Maßstab entgegenstehen.

Es ist auf Grund des oben gesagten offensichtlich, daß die aufgezeigten C-N-Kupplungsverfahren einerseits prinzipiell gut zur Darstellung entsprechender Arylamine geeignet sind, andererseits die bisherigen Methoden aber für bestimmte Belange (mehrere Reaktionszentren, multifunktionell) noch unzureichend sind. Dementsprechend besteht ein deutlicher Bedarf an Verbesserung des oben gezeigten Stands der Technik.

Es wurde nun überraschend gefunden, daß die oben im Stand der Technik aufgezeigte C-N-Kupplungsreaktion durch eine klar definierte Änderung entscheidend verbessert werden kann, so daß die oben aufgezeigten Probleme gelöst werden können.

Das neue, erfindungsgemäße Verfahren beschreibt ein Verfahren zur Herstellung tertiärer Amine der Formel (I)

Ar-(NR1R2)ₙ (I)

durch Umsetzung sekundärer Amine der Formel (II)

H-NR1R2 (II)

mit Aromaten oder Heteroaromaten der Formel (III)

(X)ₙ-Ar (III)

in Gegenwart einer Base und
einer Nickel- oder Palladiumverbindung und
eines oder mehrerer Phosphin(e) ausgewählt aus der Gruppe der monomeren, oligomeren und/oder polymeren Halogen-Phosphine der allgemeinen Formel Y-PR4R5, Dihalogen-Phosphine der allgemeinen Formel (Y)₂PR4, Alkoxy- und/oder Aryloxy-Phosphine der allgemeinen Formel R3O-PR4R5, Dialkoxy- und/oder Diaryloxy-phosphine der allgemeinen Formel (R3O)₂-PR4, sowie Mischungen derselben untereinander, in einem inerten Lösungsmittel.

Aromaten- bzw. Heteroaromaten (X)ₙ-Ar im Sinne dieser Anmeldung sind Aromaten mit 6 bis 40 C-Atomen und/oder Heteroaromaten mit 2 bis 40 C-Atomen, welche beide mit einem oder auch mehreren linearen Alkyl- bzw. Alkoxyresten mit 1 bis 20 C-Atomen oder verzweigten oder cyclischen Alkyl- bzw. Alkoxyresten mit bis zu 20 C-Atomen, bei denen wiederum eine oder mehrere CH₂-Gruppen, die nicht aufeinander folgen, durch O, S, C=O, oder eine Carboxygruppe ersetzt sein können, unsubstituierten C-4 bis C-20 Aryl- oder Heteroarylresten, Fluor, Cyano, Nitro substituiert sein können bzw. auch unsubstituiert sein können. Verbindungen, die bevorzugt verwendet werden können, sind die entsprechenden substituierten oder unsubstituierten Derivate von Benzol, Naphthalin, Anthracen, Pyren, Biphenyl, Fluoren, Spiro-9,9'-bifluoren, Phenanthren, Triptycen, Pyridin, Furan, Thiophen, Pyrrol, Chinolin, Chinoxalin, Pyrimidin und Pyrazin.

Monofunktionelle Aromaten zeichnen sich durch n = 1 aus, polyfunktionelle durch n > 1, bevorzugt aber n kleiner gleich 10. Bevorzugt steht n für eine ganze Zahl 1, 2, 3, 4, 5, 6, 7, 8, 9 oder 10, insbesondere bevorzugt für eine ganze Zahl 1, 2, 3, 4, 5 oder 6.

Die funktionelle Gruppe X in den Aryl- oder Heteroarylverbindungen ist eine für die beschriebene Umsetzung geeignete reaktive Abgangsgruppe, ausgewählt aus Chlor, Brom, lod, Methylsulfonat, Tosylat, Triflat, Nonaflat bzw. einer Diazoniumsalz-Gruppierung.

Bei den erfindungsgemäß eingesetzten sekundären Aminen des Typs H-NR1 R2 sind die Reste R1 und R2 bei jedem Auftreten gleich oder verschieden und stehen für einen linearen Aliphaten mit 1 bis 20 C-Atomen oder einen verzweigten, mono-, bi-, tri- oder poly-cyclischen Aliphaten mit bis zu 20 C-Atomen, wobei der Stickstoff Bestandteil des Ringsystems sein kann, und bei denen wiederum eine oder mehrere CH₂-Gruppen, die nicht aufeinander folgen, durch NR4, O, S, C=O oder eine Carboxygruppe ersetzt sein können,
und Aromaten mit 6 bis 40 C-Atomen bzw. Heteroaromaten mit 2 bis 40 C-Atomen, welche beide mit einem oder auch mehreren linearen Alkyl- bzw. Alkoxyresten mit 1 bis 20 C-Atomen oder verzweigten oder cyclischen Alkyl- bzw. Alkoxyresten mit bis zu 20 C-Atomen, bei denen wiederum eine oder mehrere CH₂-Gruppen, die nicht aufeinander folgen, durch O S, C=O oder eine Carboxygruppe ersetzt sein können, unsubstituierten C-4 bis C-20 Aryl- oder Heteroarylresten, Fluor, Cyano, Nitro substituiert sein können bzw. auch unsubstituiert sein können.

Bevorzugte Alkylreste bei R1 und R2 sind Methyl-, Ethyl-, Propyl-, *iso*-Propyl-, Butyl-, *iso*-Butyl-, *sec*-Butyl-, *tert*-Butyl-, Cyclopentyl-, Cyclohexyl-, Cycloheptyl-, N-Methylpiperazinyl- und Morpholino-Reste.

Bevorzugte Aryl- bzw. Heteroarylrestereste bei R1 und R2 sind die entsprechenden substituierten oder unsubstituierten Derivate von Benzol, Naphthalin, Anthracen, Pyren, Biphenyl, Fluoren, Spiro-9,9'-bifluoren, Phenanthren, Triptycen, Pyridin, Furan, Thiophen, Pyrrol, Chinolin, Chinoxalin, Pyrimidin und Pyrazin.

Die Basen werden analog den Anmeldungen US 5576460 und EP-A-0 802 173 verwendet. Es werden sowohl anorganische Basen, insbesondere Alkali- und Erdalkalimetallcarboxylate, -carbonate, -hydrogencarbonate und -phosphate wie Natrium- und Kaliumacetat, -carbonat und -phosphat eingesetzt als auch organische Basen, insbesondere Metall-Alkoholate des Typs MO-R3, wobei M ein elektropositives Metall, bevorzugt Lithium-, Natrium-, Kalium-, Magnesium- und Zinkist, und R3 ein einfacher linearer Alkyl-Rest mit 1 bis 12 C-Atomen oder ein verzweigter Alkyl-Rest mit bis zu 12 C-Atomen, bevorzugt Methyl-, Ethyl-, Propyl-, *iso*-Propyl-, Butyl-, *sec*-Butyl, *iso*-Butyl-, *tert*-Butyl-, *tert*-Pentyl-, *tert*-Hexyl- oder ein einfacher Aryl-Rest mit 6 bis 12 C-Atomen, der bevorzugt Phenyl ist, verwendet. Es kann bevorzugt sein, oligomere oder polymere Metall-Alkoholate zu verwenden, wobei in diesen Fällen R3 einem oligomeren oder polymeren Alkohol im weitesten Sinne entspricht.
Besonders bevorzugt wird als Base das Metall-Alkoholat Natrium-*tert*-butanolat verwendet. Gegebenenfalls können Mischungen der Basen verwendet werden.

Bei dem eingesetzten monomeren, oligomeren und/oder polymeren Phosphin handelt es sich um eine oder mehrere Verbindungen ausgewählt aus der Gruppe der Halogen-Phosphine des Typs Y-PR4R5, der Dihalogen-Phosphine des Typs (Y)₂-PR4, der Alkoxy- und/oder Aryloxy-Phosphine des Typs R30-PR4R5, der Dialkoxy- und/oder Diaryloxy-Phosphine des Typs (R3O)₂-PR4, sowie Mischungen derselben untereinander.
Die Reste R4 und R5 sind bei jedem Auftreten gleich oder verschieden und stehen für einen linearen Alkyl-Rest mit 1 bis 12 C-Atomen oder einen verzweigten oder mono-, di- oder tricyclischen Alkyl-Rest mit bis zu 12 C-Atomen, bei denen wiederum eine oder mehrere CH₂-Gruppen, die nicht aufeinander folgen, durch O ersetzt sein können, bzw. einem Aryl- bzw. Heteroaryl-Rest mit 4 bis 12 C-Atomen, welche mit einem oder auch mehreren linearen Alkyl- bzw. Alkoxyresten mit 1 bis 10 C-Atomen oder verzweigten oder cyclischen Alkyl- bzw. Alkoxyresten mit bis zu 10 C-Atomen, bei denen wiederum eine oder mehrere CH₂-Gruppen, die nicht aufeinander folgen, durch O, S, C=O oder eine Carboxygruppe ersetzt sein können, substituiert sein können.
Innerhalb der Alkylreste R4 und R5 sind Methyl-, Ethyl-, Propyl-, *iso*-Propyl-, Butyl-, *iso*-Butyl-, *sec*-Butyl-, *tert-*Butyl-, *tert-*Pentyl-, *tert*-Hexyl-, Cyclopentyl- und Cyclohexyl-Reste bevorzugt.

Innerhalb der Aryl- bzw. Heteroarylreste R4 und R5 sind Phenyl-, o-Tolyl-, 2,6-Dimethylphenyl-, Mesityl-, 2-*lso*-propylphenyl-, 2,6-Di-*iso*-propylphenyl-, 2-*Tert-*butylphenyl-, 2-Methoxyphenyl, 2,6-Dimethoxyphenyl-, 2,4,6-Trimethoxyphenyl und 2-Biphenyl-Rest bevorzugt.

Es kann bevorzugt sein, statt der oben beschriebenen monomeren Halogen-, Dihalogen-, Alkoxy-, Aryloxy-, Dialkoxy- und/oder Diaryloxy-Phosphine oligomer oder polymer geträgerte Halogen-, Dihalogen-, Alkoxy-, Aryloxy-, Dialkoxy- und/oder Diaryloxy-Phosphine gemäß Schema 4 einzusetzen, wobei in diesen Fällen die Reste R4 und/oder R5 einer oligomeren oder polymeren Matrix im weitesten Sinne entsprechen können.

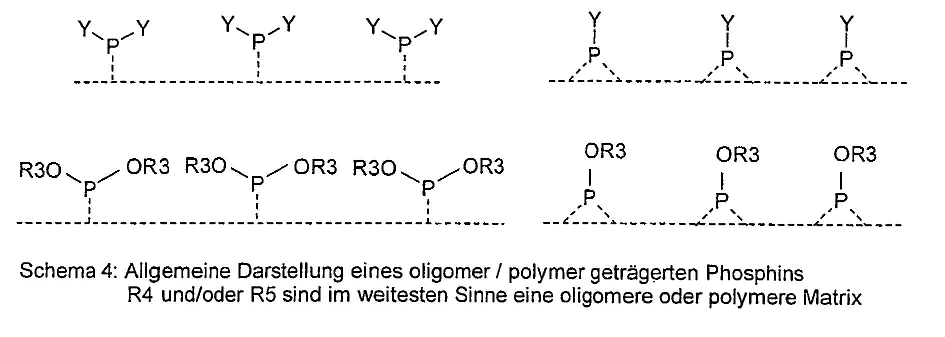

Es kann auch bevorzugt sein, statt der oben beschriebenen monomeren Alkoxy-, Aryloxy-, Dialkoxy- und/oder Diaryloxy-Phosphine oligomer oder polymer geträgerte Alkoxy-, Aryloxy-, Dialkoxy- und/oder Diaryloxy-Phosphine gemäß Schema 5 einzusetzen, wobei in diesen Fällen R3 einem oligomeren oder polymeren Alkohol im weitesten Sinne entspricht.

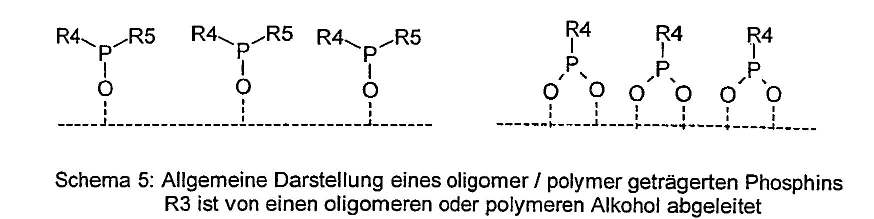

Die Gruppe Y steht für Halogen, vorzugsweise Chlor oder Brom.

Das hier beschriebene Verfahren ist dadurch gekennzeichnet, daß als Phosphin ein Halogen-Phosphin des Typs Y-PR4R5 bzw. ein Dihalogen-Phosphin des Typs Y₂-PR4 eingesetzt werden kann, es ist aber wie oben beschrieben nicht darauf beschränkt.

Unter den Reaktionsbedingungen unterliegen die Halogen-Phosphine bzw. die Dihalogen-Phosphine möglicherweise in Situ, durch salzmetathetische Reaktion mit der zugesetzten Base (Metall-Alkoholat, MO-R3), einer Umwandlung zu Alkoxy-, Aryloxy-, Dialkoxy- bzw. Diaryloxy-Phosphinen, gemäß Schema 6:

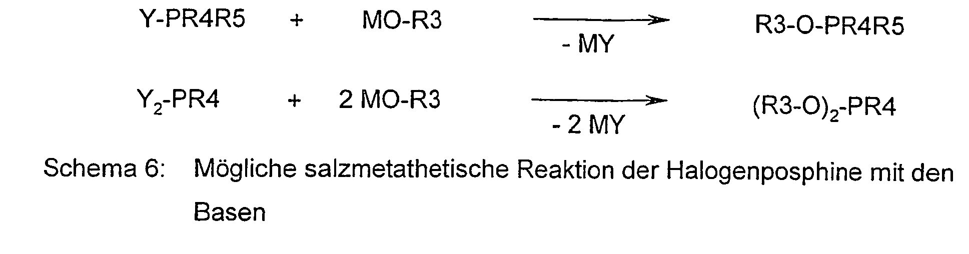

Derartige Alkoxy-, Aryloxy-, Dialkoxy- bzw. Diaryloxy-Phosphine sind ebenfalls erfindungsgemäß und Bestandteil dieser Beschreibung, unabhängig davon, ob sie in Situ im Reaktionsgemisch gebildet werden, sie vorab durch Umsetzung eines Halogen-Phosphins des Typs Y-PR4R5 oder eines Dihalogen-Phosphins des Typs Y₂-PR4 mit einem Metall-Alkoholat MO-R3 erzeugt und dann dem Reaktionsgemisch zugesetzt werden, oder ob sie als solche in Reinsubstanz eingesetzt werden.

Bevorzugt werden als Halogen-Phosphine Chlor-di(*iso*-propyl)phosphin, Chlor-di(iso-butyl)phosphin, Chlor-di(*tert*-butyl)phosphin, Chlor-di(cyclohexyl)phosphin, Chlor-di(o-tolyl)phosphin, Chlor-di(mesityl)phosphin, Chlor-bis(2-methoxyphenyl)phosphin, Chlor-bis(2,4,6-trimethoxyphenyl)phosphin bzw. die analogen / abgeleiteten Alkyloxy- bzw. Aryloxy-Phosphine und als Dihalogen-Phosphine Dichlor-isopropylphosphin, Dichlor-*iso*-butylphosphin, Dichlor-*tert*-butylphosphin, Dichlorcyclohexylphosphin, Dichlor-o-tolylphosphin, Dichlor-mesitylphosphin, Dichlor-2-methoxyphenylphosphin, Dichlor-2,4,6-trimethoxyphenylphosphin bzw. die analogen / abgeleiteten Dialkyloxy- bzw. Diaryloxy-Phosphine verwendet.

Besonders bevorzugte verwendete Halogen-Phosphine sind Chlor-di(iso-propyl)phosphin, Chlor-di(*tert*-butyl)phosphin und Chlor-bis(2,4,6-trimethoxyphenyl) phosphin bzw. die analogen / abgeleiteten Alkyloxy- bzw. Aryloxy-Phosphine und besonders bevorzugte verwendete Dihalogen-Phosphine sind Dichlor-isopropylphosphin, Dichlor-*tert*-butylphosphin, Dichlor-2,4,6-trimethoxyphenylphosphin bzw. die analogen / abgeleiteten Dialkyloxy- bzw. Diaryloxy-Phosphine.

Die erfindungsgemäße Nickelverbindung bedeutet eine Nickel-(II)-verbindungen, bevorzugt Nickel-(II)-halogenide, Nickel-(II)-biscarboxylate, Nickel-(II)-ketonate, oder daraus ableitbare Komplexe wie Olefinnickel-(II)-halogenide, Allyinickel-(II)-halogenide bzw. auch disperses oder kolloidales metallisches Nickel geträgert oder ungeträgert oder auch weitere Nickel-(0)-verbindungen.
Die erfindungsgemäße Palladiumverbindung bedeutet eine Palladium-(II)-verbindungen, bevorzugt Palladium-(II)-halogenide, Palladium-(II)-biscarboxylate, Palladium-(II)-ketonate, oder einfache daraus ableitbare Komplexe wie Nitrilpalladium-(II)-halogenide, Olefinpalladium-(II)-halogenide, Allylpalladium-(II)-halogenide bzw. auch disperses oder kolloidales metallisches Palladium geträgert oder ungeträgert oder auch weitere Palladium-(0)-verbindungen.
Besonders bevorzugt werden als Palladiumverbindung Palladium-(II)-acetat oder Tris-(dibenzylidenaceton)-dipalladium (0) (Pd₂(dba)₃ bzw. Pd₂(dba)₃ x CHCl₃) verwendet werden.

Als Lösemittel werden inerte Lösemittel verwendet, d. h. bevorzugt aprotische Lösemittel, besonders bevorzugt aromatische Lösungsmittel wie Toluol, die isomeren Xylole, beziehungsweise Mischungen dieser, Ether, cyclische Ether und Polyether wie Di-*iso*-propylether, Methyl-*tert*-butylether, Di-n-butylether, Anisol, Tetrahydrofuran, Dioxan, Di-, Tri- und Tetraethylenglykoldimethyl- bzw. ethylether und Oligo- bzw. Polyethylenglykoldimethyl- bzw. ethylether, N,N-Dialkylcarbonsäureamide und N-Alkyllactone wie Dimethylformamid, Dimethylacetamid oder N-Methylpyrrolidin-2-on. Gegebenenfalls werden auch Mischungen dieser Lösemittel eingesetzt.

Bei dem erfindungsgemäßen Verfahren wird die Nickel-oder Pällädiumverbindung in der Regel in einer Menge von 0.00001 mol % bis 10 mol% (Nickel bzw. Palladium) bezogen auf die Menge zu schließender C-N-Verknüpfungen eingesetzt. Bevorzugt ist hier der Bereich von 0.001 % bis 5%, besonders bevorzugt der Bereich von 0.01 % bis 2.5%.

Bei dem erfindungsgemäßen Verfahren beträgt das molare Verhältnis der Nickel- bzw. Palladiumverbindung zum Halogen-Phosphin der allgemeinen Formel Y-PR4R5, zum Dihalogen-Phosphin der allgemeinen Formel Y₂PR4, zum Alkoxy- bzw. Aryloxy-Phosphin der allgemeinen Formel R3O-PR4R5 oder zum Dialkoxy- bzw. Diaryloxy-phosphins der allgemeinen Formel (R3O)₂-PR4 zwischen 0.5 bis 20 und bevorzugt 1 bis 8.
Die Base, insbesondere das Metallalkoholat M-OR3, wird in der Regel zwischen 0.5 und 10 Äquivalenten, bezogen auf die Menge zu schließender C-N-Verknüpfungen eingesetzt. Bevorzugt ist hier der Bereich zwischen 0.8 und 5 Äquivalenten, besonders bevorzugt zwischen 1.0 bis 3 Äquivalenten.

Die Konzentration der Reaktanden im Lösemittel hängt natürlich von der speziellen Reaktion ab. Bevorzugt findet die Reaktion jedoch im Bereich von 0.1 mol/l bis zu 5 mol/l bezogen auf die Menge zu schließender C-N-Verknüpfungen statt.

Die erfindungsgemäße Reaktion ist thermisch aktiviert und findet daher in der Regel im Temperaturbereich oberhalb Raumtemperatur statt, bevorzugt von 40° bis 200°C, besonders bevorzugt von 60° bis 180°C, ganz besonders bevorzugt von 80° bis 160°C.

Die erfindungsgemäße Reaktion läuft in der Regel in 10 Minuten bis zu 48 h (Stunden), bevorzugt in 1 h bis zu 6 h, ab.

Gegebenenfalls können zur Homogenisierung des Reaktionsgemischs inerte Mahlkörper z.B. Metall-, Glas-, Keramikkugeln oder Raschig- oder Pallringe zugesetzt werden.

Das erfindungsgemäße Verfahren ist für die Darstellung von definierten Verbindungen bestens geeignet ohne jedoch auf die beschränkt zu sein. Bei Verwendung geeigneter Edukte, wie z.B. Diamine oder Di-Halogenarylene, kann es auch zur Synthese oligomerer oder polymerer Verbindungen eingesetzt werden. Das erfindungsgemäße Verfahren zeigt folgende überraschende Vorteile:
- Die verwendeten Halogen-, Dihalogen-, Alkyloxy-, Aryloxy-, Dialkyloxy-, bzw. Dialkyloxy-Phosphine zeigen bei Exposition an Luft kein pyrophores Verhalten, sie sind je nach organischem Rest zum Teil gegenüber atmosphärischem Sauerstoff und Wasser stabil. Dies ist insbesondere bei einem technischen Einsatz in Explosionsschutzzonen von erheblichem Vorteil gegenüber bekannten Verfahren.
- Die verwendeten Halogen-, Dihalogen-, Alkoxy-, Aryloxy-, Dialkoxy-, bzw. Dialkoxy-Phosphine sind zum Teil nach einfachen Literatur-bekannten Verfahren kostengünstig darstellbar oder aber zum Teil auch kommerziell erhältlich.
- Aufgrund der hohen Umsätze werden hohe Ausbeuten an Rohprodukten erreicht.
- Bedingt durch den geringen Anteil an Nebenprodukten, insbesondere durch den geringen Anteil durch Destillation und / oder Kristallisation nur schwer abtrennbarer Regio-Isomere, ist die Ausbeute an Produkten mit einer Reinheit > 99.5 % bzw. hochreiner Produkte mit einer Reinheit von > 99.9 % deutlich größer als bei Verwendung bereits bekannter Verfahren (s. Tabelle 1, Experimenteller Teil dieser Anmeldung).

Die nach dem hier beschriebenen Verfahren erzielten Reinheiten sind mit bisher beschriebenen Verfahren nicht oder nur mit aufwendigen Reinigungsschritten zu erzielen. Verbindungen, erhalten nach dem hier beschriebenen Verfahren, weisen eine Reinheit von mehr als 99.5 % auf.

Ebenso sind die Verbindungen, erhalten nach dem hier beschriebenen Verfahren, dadurch gekennzeichnet, daß der prozentuale Anteil (mittels ¹H-NMR und/oder HPLC bestimmt) an möglichen Regio-Isomeren, die im Verlauf der C-N-Kupplung gebildet werden können, weniger als 1 % bezogen auf das erwartete Isomere beträgt.

Die beschriebene Erfindung wird durch die nachfolgend aufgeführten Beispiele erläutert, ist aber keinesfalls auf diese beschränkt, sondern kann durch den Fachmann natürlich einfach auf die oben aufgezeigten bzw. in der zitierten Literatur beschriebenen Systeme übertragen werden.

### Synthese der Arylamine

Die nachfolgenden Synthesen wurden bis zur Aufarbeitung unter einer trockenen Rein-Stickstoffatmosphäre oder -Argonatmosphäre unter Verwendung trockener Lösungsmittel durchgeführt. Die verwendeten Edukte [Natrium-*tert*-butanolat, Palladium(II)acetat, Chlor-di-*tert*-butylphosphin, Diphenylamin, 1-Naphthyl-phenylamin, Bis(4-methoxyplenyl)amin] wurden bei ALDRICH bezogen und ohne weitere Aufreinigung eingesetzt. Das 2,2',7,7'-Tetrabrom-9,9'-spirobifluoren (Reinheit > 99.7 %) wurde nach R. Wu, J. S. Schumm, D. L. Pearson, J. M. Tour, J. Org. Chem., **1996,** *61,* 6906-6921 und entsprechender Reinigung durch Umkristallisation aus Dioxan erhalten.
Die strukturelle Integrität der Produkte wurde via ¹H-NMR-Spektroskopiefestgestellt, die Reinheit der Produkte wurde mittels HPLC bestimmt.

### Beispiel 1: 2,2',7,7'-Tetrakis-(N,N'-diphenylamino)spiro-9,9'-bifluoren

Eine entgaste Suspension von 31.6 g (50 mmol) 2,2',7,7'-Tetrabrom-9,9'-spirobifluoren und 30.3 g (315 mmol) Natrium-*tert*-butanolat in 400 ml Toluol wurde mit 361 mg = 380 µl (2 mmol) Chlor-di-*tert*-butylphosphin versetzt. Nach 5 minütigem Rühren wurde die Reaktionsmischung mit 225 mg (1 mmol) Palladium(II)acetat und dann mit 38.1 g (225 mmol) Diphenylamin versetzt. Die Reaktionsmischung wurde 2 h unter Rückfluß erhitzt. Nach Abkühlen auf 60°C wurden 500 ml Wasser zugegeben und 1 h nachgerührt. Das ausgefallene Produkt wurde abfiltriert, mit Wasser gewaschen und anschließend im Vakuum bei 80°C bis zur Gewichtskonstanz getrocknet. Durch Einengen der organischen Phase auf 100 ml und Abfiltieren des ausgefallenen Niederschlags wurde eine zweite Produktfraktion erhalten.
Die vereinigten Produktfraktionen wurden aus Dioxan (2.2 ml / 1 g) unter Zusatz von wenig Hydrazinhydrat bis zur gewünschten Reinheit umkristallisiert und dann im Hochvakuum sublimiert (p = 5 x 10⁻⁵ mbar, T = 375°C) (s. Tabelle 1).
¹H-NMR (0.8 ml CDCl₃ mit 30 µl Hydrazinhydrat): 7.44 (d, ³J_{HH} = 8.2 Hz, H4, 4 H), 7.20 - 7.16 (m, 16 H), 6.99 - 6.95 (m, 24 H), 6.91 (dd, ³J_{HH} = 8.2 Hz, ⁴J_{HH} = 2.0 Hz, H3, 4 H), 6.68 (d, ⁴J_{HH} = 2.0 Hz, H1, 4 H).

### Beispiel 2:

Durchführung analog zu Beispiel 1, mit dem Unterschied, daß das 2,2',7,7'-Tetrabrom-9,9'-spirobifluoren und das Diphenylamin vorgelegt wurden, und das Natrium-*tert*-butanolat abschießend zugegeben wurde. Ausbeute / Reinheit s. Tabelle 1.
¹H-NMR: s. Beispiel 1

### Beispiel 3: Vergleichsbeispiel zu Beispiel 1 nach EP-A-0 802 173

Durchführung analog zu Beispiel 1, mit dem Unterschied, daß das Chlor-di-*tert-*butylphosphin durch 405 mg (2 mmol) Tri*-tert-*butylphosphin ersetzt wurde. Ausbeute / Reinheit s. Tabelle 1.
¹H-NMR: s. Beispiel 1

### Beispiel 4: Vergleichsbeispiel zu Beispiel 1 nach WO 01/40147

Durchführung analog zu Beispiel 1, mit dem Unterschied, daß als Phosphin Di-*tert-*butylphosphinoxid welches durch Hydrolyse von 361 mg = 380 µl (2 mmol) Chlor-di-*tert*-butylphosphin gemäß "Experiment 2 nach WO 01/40147 " dargestellt wurde, eingesetzt wurde. Die Reaktionsmischung wurde Aufgrund des langsamen Reaktionsfortgangs 24 h unter Rückfluß erhitzt. Auch nach dieser Zeit wurde kein vollständiger Umsatz des 2,2',7,7'-Tetrabrom-9,9'-spirobifluoren erreicht, s. Tabelle 1.

### Beispiel 5: 2,2',7,7'-Tetrakis-(N-phenyl-N'-(1-naphthyl)amino)spiro-9,9'-bifluoren

Eine entgaste Suspension von 31.6 g (50 mmol) 2,2',7,7'-Tetrabrom-9,9'-spirobifluoren und 30.3 g (315 mmol) Natrium-*tert*-butanolat in 400 ml Toluol wurde mit 361 mg = 380 µl (2 mmol) Chlor-di-*tert*-butylphosphin versetzt. Nach 5 minütigem Rühren wurde die Reaktionsmischung mit 225 mg (1 mmol) Palladium(II)acetat und dann mit 49.3 g (225 mmol) 1-Naphthyl-phenylamin versetzt. Die Reaktionsmischung wurde 2 h unter Rückfluß erhitzt. Nach Abkühlen auf 60°C wurden 500 ml Wasser zugegeben und 1 h nachgerührt. Die organische Phase wurde abgetrennt und bis zur Trockene eingeengt. Der Rückstand wurde in 500 ml Dichlormethan aufgenommen und unter Rühren langsam in 1000 ml Methanol eingetragen. Das so erhaltene Rohprodukt wurde abgesaugt, mit Methanol gewaschen, getrocknet, aus Dioxan (2.0 ml / 1g) unter Zusatz von wenig Hydrazinhydrat bis zur gewünschten Reinheit umkristallisiert und dann im Hochvakuum sublimiert (p = 5 x 10⁻⁵ mbar, T = 405°C). Ausbeute / Reinheit s. Tabelle 1.
¹H-NMR (0.8 ml CDCl₃ mit 30 µl Hydrazinhydrat): 7.88-7.84 (m, 8 H), 7.75-7.73 (m, 4 H), 7.46-7.41 (m, 8 H), 7.34-7.26 (m, 8 H), 7.22-7.20 (m, 4 H), 7.12-7.08 (m, 8 H), 6.86-6.80 (m, 16 H), 6.77-6.74 (m, 4 H).

### Beispiel 6: Vergleichsbeispiel zu Beispiel 5 nach EP-A-0 802 173

Durchführung analog zu Beispiel 4, mit dem Unterschied, daß das Chlor-di-*tert-*butylphosphin durch 405 mg (2 mmol) Tri-*tert*-butylphosphin ersetzt wurde. Ausbeute / Reinheit s. Tabelle 1.

### Beispiel 7: 2,2',7,7'-Tetrakis-(N,N'-di(4-methoxy)phenylamino)spiro-9,9'-bifluoren

Eine entgaste Suspension von 31.6 g (50 mmol) 2,2',7,7'-Tetrabrom-9,9'-spirobifluoren und 30.3 g (315 mmol) Natrium-*tert*-butanolat in 400 ml Toluol wurde mit 361 mg = 380 µl (2 mmol) Chlor-di-*tert*-butylphosphin versetzt. Nach 5 minütigem Rühren wurde die Reaktionsmischung mit 225 mg (1 mmol) Palladium(II)acetat und dann mit 51.6 g (225 mmol) Bis(4-methoxyphenyl)amin versetzt. Die Reaktionsmischung wurde 2 h unter Rückfluß erhitzt. Nach Abkühlen auf 60°C wurden 500 ml Wasser zugegeben und 1 h nachgerührt. Die organische Phase wurde abgetrennt und bis zur Trockene eingegengt. Der Rückstand wurde in 500 ml Dichlormethan aufgenommen und unter Rühren langsam in 1000 ml Methanol eingetragen. Der so erhaltene Rohprodukt wurde abgesaugt, mit Methanol gewaschen, getrocknet, aus Dioxan (1.6 g / 1 ml) unter Zusatz von Hydrazinhydrat bis zur gewünschten Reinheit umkristallisiert und anschließend im Hochvakuum unter Schmelzen getrocknet. Ausbeute und Reinheit s. Tabelle 1.
¹H-NMR (0.8 ml CDCl₃ mit 30 µl Hydrazinhydrat): 7.35 (d, ³J_{HH} = 10.4 Hz, H4, 4 H), 6.92 - 6.89 (m, 16 H), 6.79 (dd, ³J_{HH} = 10.4 Hz, ⁴J_{HH} = 2.5 Hz, H3, 4 H), 6.77 - 6.73 (m, 16 H), 6.54 (d, ⁴J_{HH} = 2.5 Hz, H1, 4 H), 3.69 (s, CH₃, 24 H).

### Beispiel 8: Vergleichsbeispiel zu Beispiel 7 nach EP-A-0 802 173

Durchführung analog zu Beispiel 6, mit dem Unterschied, daß das Chlor-di-*tert-*butylphosphin durch 405 mg (2 mmol) Tri-*tert*-butylphosphin ersetzt wurde. Ausbeute / Reinheit s. Tabelle 1.

### Beispiel 9: Synthese von 4,4'-Bis(4-tert-butylphenylphenylamino)biphenyl

In einen 4 1 Stickstoffkolben wurden 2 1 Xylol unter Stickstoff entgast. Diese wurden mit 86.5 g (0.90 mol) Natrium-tert-butanolat und dann unter gutem Rühren während 5 min. mit 85.0 ml (0.45 mol) Chlor-di-tert-butylphoshin versetzt.
In einem 500 I Kessel mit Ankerrüher wurden 10.0 kg (29.7 mol) N,N'-Diphenylbenzidin in 170 | Xy|o| bei Raumtemperatur vorgelegt. Unter gutem Rühren wurde der Kessel dreimal auf einen Innendruck von ca. 50 mbar evakuiert und mit trockenem Reinstickstoff (99.998 % ig) geflutet. Die so inertisierte Lösung wurde mit 8.7 kg (90.5 mol) festem Natrium-tert-butanolat versetzt und 30 min. bei Raumtemperatur gerührt.
Anschließend wurde die Mischung aus Chlor-di-tert-butylphoshin und Natrium-tert-butanolat in 2 I Xylol zugegeben und 15 min. nachgerührt. Dann wurden 33.5 g (0.15 mol) Palladium(II)acetat zugefügt und 15 min. nachgerührt. Anschließend wurden 13.9 kg (65.2 mol) 1-Brom-4-tert-Butylbenzol zugesetzt. Die Reaktionsmischung wurde unter gutem Rühren 3 h unter Rückfluß erhitzt. Nach Abkühlen auf 60°C wurde mit 75 1 E-Wasser versetzt und weiter 3 h bei Raumtemperatur nachgerührt. Er ausgefallene Feststoff wurde über eine Drucknutsche abgesaugt und anschießend dreimal mit je 75 IE-Wasser und zweimal mit je 75 1 Ethanol ausgerührt. Nach Trockenblasen mit Stickstoff wurde der Filterkuchen unter reduziertem Druck im Trockenschrank getrocknet.
Ausbeute: 11.0 kg (18.3 mol), 61.6 % der Theorie.
Reinheit: 99.8% (HPLC), Regioisomere < 0.1 % (HPLC).

**Tabelle 1: Ausbeuten und Zusammensetzung der Produkte aus den Beispielen 1-8**

| **Bsp.** | **Ausbeute Rohprodukt** | **Reinheit Rohprodukt** | **Regio-Isomer 1** | **Regio-Isomer 2** | **Zahl der Umkistallisationen** | **Endausbeute; Reinheit** |
|---|---|---|---|---|---|---|
| **1** | **48.2 g = 97.7 %** | **98.81 %** | **0.26 %** | **0.09 %** | **3** | **83.4 %; > 99.9 %** |
| **2** | **47.9 g =97.3 %** | **98.64%** | **0.27%** | **0.09%** | **4** | **79.8 %;>99.9 %** |
| 3 | 45.8g=93.1% | 96.44% | 0.71% | 0.25% | 9 | 67.3%;>99.9% |
| 4 | Reaktionsmischung enthielt ca. 56 % des Produkts, Aufarbeitung wurde nicht durchgeführt | | | | | |
| **5** | **56.4 g = 95.2 %** | **95.97 %** | **0.86 %** | **0.33 %** | **3** | **84.1 %; > 99.5 %** |
| 6 | 52.2g=88.0% | 96.31% | 1.58% | 0.49% | 7 | 57.9%;>99.5% |
| **7** | **57.9 g = 94.6 %** | **98.06%** | **0.38%** | **0.08%** | **2** | **76.3%;>99.9%** |
| 8 | 48.3g=83.5% | 97.10% | 1.19% | 0.29% | 5 | 51.9%;>99.9% |

## Patentansprüche

1. Verfahren zur Herstellung tertiärer Amine der Formel (I)
Ar-(NR1R2)ₙ (I)
durch Umsetzung sekundärer Amine der Formel (II)
H-NR1R2 (II)
mit Aromaten oder Heteroaromaten der Formel (III)
(X)ₙ-Ar (III)
in Gegenwart einer Base und
einer Nickel- oder Palladiumverbindung und
eines oder mehrerer Phosphin(e) ausgewählt aus der Gruppe der monomeren, oligomeren und/oder polymeren Halogen-Phosphine der allgemeinen Formel Y-PR4R5, der Dihalogen-Phosphine der allgemeinen Formel (Y)₂PR4, der Alkoxy- und/oder Aryloxy-Phosphine der allgemeinen Formel R3O-PR4R5, der Dialkoxy- und/oder Diaryloxy-phosphine der allgemeinen Formel (R3O)₂-PR4, sowie Mischungen derselben untereinander, in einem inerten Lösungsmittel, wobei die Reste und Indizes folgende Bedeutung haben:
Ar sind gleich Aromaten mit 6 bis 40 C-Atomen, Heteroaromaten mit 2 bis 40 C-Atomen, welche beide mit einem oder auch mehreren linearen Alkyl- bzw. Alkoxyresten mit 1 bis 20 C-Atomen, einem oder auch mehreren verzweigten oder cyclischen Alkyl- bzw. Alkoxyresten mit bis zu 20 C-Atomen, bei denen wiederum eine oder mehrere CH₂-Gruppen, die nicht aufeinander folgen, durch O, S, C=O oder eine Carboxygruppe ersetzt sein können, unsubstituierten C-4 bis C-20 Aryl- oder Heteroarylresten, Fluor, Cyano, Nitro substituiert sein können oder auch unsubstituiert sein können,
R1, R2 sind gleich oder verschieden bei jedem Auftreten ein linearer Aliphat mit 1 bis 20 C-Atomen oder ein verzweigter, mono-, bi-, tri- oder poly-cyclischer Aliphat mit bis zu 20 C-Atomen, wobei der Stickstoff Bestandteil des Ringsystems sein kann und bei denen wiederum eine oder mehrere CH₂-Gruppen, die nicht aufeinander folgen, durch NR4, O, S, C=O oder eine Carboxygruppe ersetzt sein können, und Aromaten mit 6 bis 40 C-Atomen bzw. Heteroaromaten mit 2 bis 40 C-Atomen, welche mit einem oder auch mehreren linearen Alkyl bzw. Alkoxyresten mit 1 bis 20 C-Atomen oder einem oder auch mehreren verzweigten oder cyclischen Alkyl- bzw. Alkoxyresten mit bis zu 20 C-Atomen, bei denen wiederum eine oder mehrere CH₂-Gruppen, die nicht aufeinander folgen, durch O, S, C=O oder eine Carboxygruppe ersetzt sein können, unsubstituierten C-4 bis C-20 Aryl- oder Heteroarylresten, Fluor, Cyano, Nitro substituiert sein können bzw. auch unsubstituiert sein können,
R3 ist ein linearer Alkyl-Rest mit 1 bis 12 C-Atomen, ein verzweigter Alkyl-Rest mit bis zu 12 C-Atomen oder ein Aryl-Rest mit 6 bis 12 C-Atomen,
R4, R5 sind gleich oder verschieden bei jedem Auftreten einem linearen Alkyl-Rest mit 1 bis 12 C-Atomen oder einem verzweigten oder mono-, di- oder tricyclischen Alkyl-Rest mit bis zu 12 C-Atomen, bei denen wiederum eine oder mehrere CH₂-Gruppen, die nicht aufeinander folgen, durch O ersetzt sein können, bzw. einem Aryl- bzw. Heteroaryl-Rest mit 4 bis 12 C-Atomen, welche mit einem oder auch mehreren linearen Alkyl- bzw. Alkoxyresten mit 1 bis 10 C-Atomen oder einem oder auch mehreren verzweigten oder cyclischen Alkyl- oder Alkoxyresten mit bis zu 10 C-Atomen, bei denen wiederum eine oder mehrere CH₂-Gruppen, die nicht aufeinander folgen, durch O, S, C=O oder eine Carboxygruppe ersetzt sein können, substituiert sein können,
X ist eine Abgangsgruppe Chlor, Brom, lod, Methylsulfonat, Tosylat, Triflat, Nonaflat und/oder eine Diazoniumsalz-Gruppierung,
Y ist gleich Fluor, Chlor, Brom, lod,
n ist eine ganze Zahl von 1 bis 10.

2. Verfahren gemäß Anspruch 1, **dadurch gekennzeichnet, daß** Ar ein substituiertes oder unsubstituiertes Benzol, Naphthalin, Anthracen, Pyren, Biphenyl, Fluoren, Spiro-9,9'-bifluoren, Phenanthren, Triptycen, Pyridin, Furan, Thiophen, Pyrrol, Chinolin, Chinoxalin, Pyrimidin oder Pyrazin bedeutet.

3. Verfahren gemäß Anspruch 1 und / oder 2, **dadurch gekennzeichnet, daß** die Alkyl-, Aryl- bzw. Heteroarylreste R1 und R2 Methyl-, Ethyl-, Propyl-, *iso*-Propyl-, Butyl-, *iso*-Butyl-, *sec*-Butyl-, *tert*-Butyl-, Cyclopentyl-, Cyclohexyl-, Cycloheptyl-, N-Methylpiperazinyl-, Morpholino- und und substituiertes oder unsubstituiertes Benzol, Naphthalin, Anthracen, Pyren, Biphenyl, Fluoren, Spiro-9,9'-bifluoren, Phenanthren, Triptycen, Pyridin, Furan, Thiophen, Pyrrol, Chinolin, Chinoxalin, Pyrimidin und Pyrazin bedeuten.

4. Verfahren nach einem oder mehreren der Ansprüche 1 bis 3, **dadurch gekennzeichnet, daß** als Basen anorganische Basen, organische Basen sowie auch Mischungen derselben verwendet werden.

5. Verfahren nach Anspruch 4, **dadurch gekennzeichnet, daß** als anorganische Basen Alkali- und Erdalkalimetallcaboxylate, -carbonate, - hydrogencarbonate und -phosphate und als organische Basen Metall-Alkoholate des Typs MO-R3, wobei M ein elektropositives Metall ist, verwendet werden.

6. Verfahren nach Anspruch 5, **dadurch gekennzeichnet, daß** als anorganische Basen Natrium- und Kaliumacetat, -carbonat und -phosphat verwendet werden.

7. Verfahren nach Anspruch 5, **dadurch gekennzeichnet, daß** das elektropositive Metall M Lithium, Natrium, Kalium, Magnesium oder Zink ist.

8. Verfahren gemäß einem oder mehreren der Ansprüche 1 bis 5 oder 7, **dadurch gekennzeichnet, daß** der Rest R3 Methyl-, Ethyl-,Propyl-, *iso-*Propyl-, Butyl-, *iso*-Butyl-, *sec*-Butyl, *tert*-Butyl-, *tert*-Pentyl-, *tert*-Hexyl- oder Phenyl- bedeutet.

9. Verfahren gemäß einem oder mehreren der Ansprüche 1 bis 5 oder 8, **dadurch gekennzeichnet, daß** als organische Base ein Metallalkoholat des Typs MO-R3 worin R3 Methyl-, Ethyl-,Propyl-, *iso*-Propyl-, Butyl-, *iso-*Butyl-, *sec*-Butyl, *tert*-Butyl-, *tert*-Pentyl-, *tert*-Hexyl- oder Phenylbedeutet, verwendet wird.

10. Verfahren gemäß einem oder mehreren der Ansprüche 1 bis 9, **dadurch gekennzeichnet, daß** die Alkyl-, Aryl bzw. Heteroarylreste R4 und R5 Methyl-, Ethyl-, Propyl-, *iso*-Propyl-, Butyl-, *iso*-Butyl-, *sec*-Butyl-, *tert-*Butyl-, *tert*-Pentyl-, *tert*-Hexyl-, Cyclopentyl-, Cyclohexyl- und Phenyl-, o-Tolyl-, 2,6-Dimethylphenyl-, Mesityl-, 2-*Iso*-propylphenyl-,2,6-Di-*iso-*propylphenyl-, 2-*tert*-Butylphenyl-, 2-Methoxyphenyl, 2,6-Dimethoxyphenyl-, 2,4,6-Trimethoxyphenyl, 2-Biphenyl- bedeuten.

11. Verfahren gemäß Anspruch 1 und / oder 10, **dadurch gekennzeichnet, daß** die Reste R4 und R5 dem *tert*-Butyl-Rest entsprechen.

12. Verfahren gemäß einem oder mehreren der Ansprüche 1 bis 11, **dadurch gekennzeichnet, daß** als Nickelverbindung Nickel-(II)-verbindungen, bevorzugt Nickel-(II)-halogenide, Nickel-(II)-biscarboxylate, Nickel-(II)-ketonate, oder einfache daraus ableitbare Komplexe wie Olefinnickel-(II)-halogenide, Allylnickel-(II)-halogenide bzw. auch disperses oder kolloidales metallisches Nickel geträgert oder ungeträgert oder auch weitere Nickel-(0)-verbindungen verwendet werden.

13. Verfahren gemäß Anspruch 12, **dadurch gekennzeichnet, daß** als Nickel-(II)-verbindungen Nickel-(II)-halogenide, Nickel-(II)-biscarboxylate oder Nickel-(II)-ketonate eingesetzt werden und als ableitbare Komplexe Olefinnickel-(II)-halogenide oder Allylnickel-(II)-halogenide verwendet werden.

14. Verfahren gemäß einem oder mehreren der Ansprüche 1 bis 11, **dadurch gekennzeichnet, daß** als Palladiumverbindung Palladium-(II)-verbindungen oder einfache daraus ableitbare Komplexe oder disperses oder kolloidales metallisches Palladium geträgert oder ungeträgert oder auch weitere Palladium-(0)-verbindungen verwendet werden.

15. Verfahren gemäß Anspruch 14, **dadurch gekennzeichnet, daß** als Palladium-(II)-verbindungen Palladium-(II)-halogenide, Palladium-(II)-biscarboxylate, Palladium-(II)-ketonate, oder als ableitbare Komplexe Nitrilpalladium-(II)-halogenide, Olefinpalladium-(II)-halogenide oder Allylpalladium-(II)-halogenide verwendet werden.

16. Verfahren gemäß einem oder mehreren der Ansprüche 1 bis 11, 14 und 15, **dadurch gekennzeichnet, daß** als Palladiumverbindung Palladium-(II)-acetat oder Pd₂(dba)₃ verwendet werden.

17. Verfahren gemäß einem oder mehreren der Ansprüche 1 bis 16, **dadurch gekennzeichnet, daß** als inertes Lösemittel aromatische Lösungsmittel, Ether, cyclische Ether und Polyether, Di-, Tri und Tetraethylenglykoldimethyl- oder ethylether und Oligo- oder Polyethylenglykoldimethyl- oder ethylether, N,N-Dialkylcarbonsäureamide und N-Alkyllactone, gegebenenfalls auch Mischungen dieser Lösemittel, verwendet werden.

18. Verfahren gemäß Anspruch 17, **dadurch gekennzeichnet, daß** als aromatisches Lösungsmittel Toluol und/oder die isomeren Xylole, Dimethylformamid, Dimethylacetamid oder N-Methylpyrrolidin-2-on, gegebenenfalls auch Mischungen dieser Lösemittel, verwendet werden.

19. Verfahren gemäß Anspruch 1 **dadurch gekennzeichnet, daß** durch Verwendung von Diamine oder Di-Halogenarylene als Edukte oligomere oder polymere Verbindungen synthetisiert werden.

## Claims

1. Process for the preparation of *tert*iary amines of the formula (I)
Ar-(NR1R2)ₙ (I)
by reaction of secondary amines of the formula (II)
H-NR1R2 (II)
with aromatics or heteroaromatics of the formula (III)
(X)ₙ-Ar (III)
in the presence of a base and
a nickel or palladium compound and
one or more phosphine(s) selected from the group of the monomeric, oligomeric and/or polymeric halophosphines of the general formula Y-PR4R5, the dihalophosphines of the general formula (Y)₂PR4, the alkoxy- and/or aryloxyphosphines of the general formula R30-PR4R5, the dialkoxy- and/or diaryloxyphosphines of the general formula (R3O)₂-PR4, and mixtures thereof with one another, in an inert solvent, where the radicals and indices have the following meaning:
Ar are equal to aromatics having 6 to 40 C atoms, heteroaromatics having 2 to 40 C atoms, both of which may be substituted by one or also more linear alkyl or alkoxy radicals having 1 to 20 C atoms, one or also more branched or cyclic alkyl or alkoxy radicals having up to 20 C atoms, in which in turn one or more non-consecutive CH₂ groups may be replaced by O, S, C=O or a carboxyl group, unsubstituted C-4 to C-20 aryl or heteroaryl radicals, fluorine, cyano, nitro, or may also be unsubstituted,
R1, R2 are, identically or differently on each occurrence, a linear aliphatic having 1 to 20 C atoms or a branched, mono-, di-, tri- or polycyclic aliphatic having up to 20 C atoms, where the nitrogen may be part of the ring system and in which in turn one or more non-consecutive CH₂ groups may be replaced by NR4, O, S, C=O or a carboxyl group, and aromatics having 6 to 40 C atoms or heteroaromatics having 2 to 40 C atoms, which may be substituted by one or also more linear alkyl or alkoxy radicals having 1 to 20 C atoms or one or also more branched or cyclic alkyl or alkoxy radicals having up to 20 C atoms, in which in turn one or more non-consecutive CH₂ groups may be replaced by O, S, C=O or a carboxyl group, unsubstituted C-4 to C-20 aryl or heteroaryl radicals, fluorine, cyano, nitro, or may also be unsubstituted,
R3 is a linear alkyl radical having 1 to 12 C atoms, a branched alkyl radical having up to 12 C atoms or an aryl radical having 6 to 12 C atoms,
R4, R5 are, identically or differently on each occurrence, a linear alkyl radical having 1 to 12 C atoms or a branched or mono-, di- or tricyclic alkyl radical having up to 12 C atoms, in which in turn one or more non-consecutive CH₂ groups may be replaced by O, or an aryl or heteroaryl radical having 4 to 12 C atoms, which may be substituted by one or also more linear alkyl or alkoxy radicals having 1 to 10 C atoms or one or also more branched or cyclic alkyl or alkoxy radicals having up to 10 C atoms, in which in turn one or more non-consecutive CH₂ groups may be replaced by O, S, C=O or a carboxyl group,
X is a leaving group chlorine, bromine, iodine, methylsulfonate, tosylate, triflate, nonaflate and/or a diazonium salt group,
Y is equal to fluorine, chlorine, bromine, iodine,
n is an integer from 1 to 10.

2. Process according to Claim 1, **characterised in that** Ar denotes a substituted or unsubstituted benzene, naphthalene, anthracene, pyrene, biphenyl, fluorene, spiro-9,9'-bifluorene, phenanthrene, triptycene, pyridine, furan, thiophene, pyrrole, quinoline, quinoxaline, pyrimidine or pyrazine.

3. Process according to Claim 1 and/or 2, **characterised in that** the alkyl, aryl or heteroaryl radicals R1 and R2 denote methyl, ethyl, propyl, *iso*-propyl, butyl, *iso*-butyl, *sec*-butyl, *tert*-butyl, cyclopentyl, cyclohexyl, cycloheptyl, N-methylpiperazinyl, morpholino and substituted or unsubstituted benzene, naphthalene, anthracene, pyrene, biphenyl, fluorene, spiro-9,9'-bifluorene, phenanthrene, triptycene; pyridine, furan, thiophene, pyrrole, quinoline, quinoxaline, pyrimidine and pyrazine.

4. Process according to one or more of Claims 1 to 3, **characterised in that** the bases used are inorganic bases, organic bases and also mixtures thereof.

5. Process according to Claim 4, **characterised in that** the inorganic bases used are alkali and alkaline earth metal carboxylates, carbonates, hydrogencarbonates and phosphates, and the organic bases used are metal alkoxides of the MO-R3 type, where M is an electropositive metal.

6. Process according to Claim 5, **characterised in that** the inorganic bases used are sodium acetate, carbonate and phosphate and potassium acetate, carbonate and phosphate.

7. Process according to Claim 5, **characterised in that** the electropositive metal M is lithium, sodium, potassium, magnesium or zinc.

8. Process according to one or more of Claims 1 to 5 or 7, **characterised in that** the radical R3 denotes methyl, ethyl, propyl, *iso*-propyl, butyl, *iso*-butyl, *sec*-butyl, *tert*-butyl, *tert*-pentyl, *tert*-hexyl or phenyl.

9. Process according to one or more of Claims 1 to 5 or 8, **characterised in that** the organic base used is a metal alkoxide of the MO-R3 type, in which R3 denotes methyl, ethyl, propyl, *iso*-propyl, butyl, *iso-*butyl, *sec*-butyl, *tert*-butyl, *tert*-pentyl, *tert*-hexyl or phenyl.

10. Process according to one or more of Claims 1 to 9, **characterised in that** the alkyl, aryl or heteroaryl radicals R4 and R5 denote methyl, ethyl, propyl, *iso*-propyl, butyl, *iso*-butyl, *sec*-butyl, *tert*-butyl, *tert-*pentyl, *tert*-hexyl, cyclopentyl, cyclohexyl and phenyl, o-tolyl, 2,6-dimethylphenyl, mesityl, 2-*iso-*propylphenyl, 2,6-di-*iso*-propylphenyl, 2-*tert*-butylphenyl, 2-methoxyphenyl, 2,6-dimethoxyphenyl, 2,4,6-trimethoxyphenyl, 2-biphenyl.

11. Process according to Claim 1 and/or 10, **characterised in that** the radicals R4 and R5 correspond to the *tert*-butyl radical.

12. Process according to one or more of Claims 1 to 11, **characterised in that** as nickel compound use is made of nickel(II) compounds, preferably nickel(II) halides, nickel(II) biscarboxylates, nickel(II) ketonates, or simple complexes which can be derived therefrom, such as olefin nickel(II) halides, allyl nickel(II) halides or also disperse or colloidal metallic nickel, supported or unsupported, or also further nickel(0) compounds.

13. Process according to Claim 12, **characterised in that** the nickel(II) compounds employed are nickel(II) halides, nickel(II) biscarboxylates or nickel(II) ketonates, and the derivable complexes used are olefin nickel(II) halides or allyl nickel(II) halides.

14. Process according to one or more of Claims 1 to 11, **characterised in that** as palladium compound use is made of palladium(II) compounds or simple complexes which can be derived therefrom or disperse or colloidal metallic palladium, supported or unsupported, or also further palladium(0) compounds.

15. Process according to Claim 14, **characterised in that** the palladium-(II) compounds used are palladium(II) halides, palladium(II) biscarboxylates, palladium(II) ketonates, or the derivable complexes used are nitrile palladium(II) halides, olefin palladium(II) halides or allyl palladium(II) halides.

16. Process according to one or more of Claims 1 to 11, 14 and 15, .. **characterised in that** the palladium compound used is palladium(II) acetate or Pd₂(dba)₃.

17. Process according to one or more of Claims 1 to 16, **characterised in that** as inert solvent use is made of aromatic solvents, ethers, cyclic ethers and polyethers, di-, tri- and tetraethylene glycol dimethyl or ethyl ether and oligo- or polyethylene glycol dimethyl or ethyl ether, N,N-dialkylcarboxamides and N-alkyllactones, optionally also mixtures of these solvents.

18. Process according to Claim 17, **characterised in that** the aromatic solvent used is toluene and/or the isomeric xylenes, dimethylformamide, dimethylacetamide or N-methylpyrrolidin-2-one, optionally also mixtures of these solvents.

19. Process according to Claim 1, **characterised in that** the use of diamines or dihaloarylenes as starting materials enables the synthesis of oligomeric or polymeric compounds.

## Revendications

1. Procédé pour la préparation d'amines tertiaires de la formule (I)
Ar-(NR1 R2)ₙ (I)
par réaction d'amines secondaires de la formule (II)
H-NR1 R2 (II)
avec des aromatiques ou des hétéroaromatiques de la formule (III)
(X)ₙ-Ar (III)
en présence d'une base et
d'un composé de nickel ou de palladium et
d'une ou de plusieurs phosphines choisies parmi le groupe des halophosphines monomériques, oligomériques et/ou polymériques de la formule générale Y-PR4RS, des dihalophosphines de la formule générale (Y)₂PR4, des alkoxy- et/ou aryloxyphosphines de la formule générale R30-PR4R5, des dialkoxy- et/ou diaryloxyphosphines de la formule générale (R3O)₂-PR4, et de mélanges de celles-ci les unes avec les autres, dans un solvant inerte, où les radicaux et les indices présentent la signification qui suit :
Ar sont équivalents à des aromatiques comportant de 6 à 40 atomes de C, à des hétéroaromatiques comportant de 2 à 40 atomes de C, dont les deux peuvent être substitués par un ou également plusieurs radicaux alkyle ou alkoxy linéaires comportant de 1 à 20 atomes de C, par un ou également plusieurs radicaux alkyle ou alkoxy ramifiés ou cycliques comportant jusqu'à 20 atomes de C, où, à leur tour, un ou plusieurs groups CH₂ non consécutifs peuvent être remplacés par O, S, C=O ou un groupe carboxyle, des radicaux aryle ou hétéroaryle C-4 à C-20 non substitués, fluor, cyano, nitro, ou peuvent également être non substitués,
R1, R2 sont, de façon identique ou différente à chaque occurrence, un aliphatique linéaire comportant de 1 à 20 atomes de C ou un aliphatique mono-, di-, tri- ou polycyclique ramifié comportant jusqu'à 20 atomes de C, où l'azote peut être une partie du système cyclique et où, à leur tour, un ou plusieurs groupes CH₂ non consécutifs peuvent être remplacés par NR4, O, S, C=O ou un groupe carboxyle, et des aromatiques comportant de 6 à 40 atomes de C ou des hétéroaromatiques comportant de 2 à 40 atomes de C, qui peuvent être substitués par un ou également plusieurs radicaux alkyle ou alkoxy linéaires comportant de 1 à 20 atomes de C ou par un ou également plusieurs radicaux alkyle ou alkoxy ramifiés ou cycliques comportant jusqu'à 20 atomes de C, où, à leur tour, un ou plusieurs groupes CH₂ non consécutifs peuvent être remplacés par O, S, C=O ou un groupe carboxyle, des radicaux aryle ou hétéroaryle C-4 à C-20 non substitués, fluor, cyano, nitro, ou peuvent également être non substitués,
R3 est un radical alkyle linéaire comportant de 1 à 12 atomes de C, un radical alkyle ramifié comportant jusqu'à 12 atomes de C ou un radical aryle comportant de 6 à 12 atomes de C,
R4, R5 sont, de façon identique ou différente à chaque occurrence, un radical alkyle linéaire comportant de 1 à 12 atomes de C ou un radical alkyle ramifié ou mono-, di- ou tricyclique comportant jusqu'à 12 atomes de C, où, à leur tour, un ou plusieurs groupes CH₂ non consécutifs peuvent être remplacés par O, ou un radical aryle ou hétéroaryle comportant de 4 à 12 atomes de C, qui peut être substitué par un ou également plusieurs radicaux alkyle ou alkoxy linéaires comportant de 1 à 10 atomes de C ou par un ou également plusieurs radicaux alkyle ou alkoxy ramifiés ou cycliques comportant jusqu'à 10 atomes de C, où, à leur tour, un ou plusieurs groupes CH₂ non consécutifs peuvent être remplacés par O, S, C=O ou un groupe carboxyle,
X est un chlore, brome, iode, méthylsulfonate, tosylate, triflate, nonaflate de groupe partant et/ou un groupe de sel de diazonium,
Y est égal à fluor, chlore, brome, iode,
n est un entier de 1 à 10.

2. Procédé selon la revendication 1, **caractérisé en ce que** Ar représente benzène, naphthalène, anthracène, pyrène, biphényle, fluorène, spiro-9,9'-bifluorène, phénanthrène, triptycène, pyridine, furane, thiophène, pyrrole, quinoline, quinoxaline, pyrimidine ou pyrazine substitué ou non substitué.

3. Procédé selon la revendication 1 et/ou 2, **caractérisé en ce que** les radicaux alkyle, aryle ou hétéroaryle R1 et R2 représentent méthyle, éthyle, propyle, *iso*-propyle, butyle, *iso*-butyle, *sec*-butyle, *tert-*butyle, cyclopentyle, cyclohexyle, cycloheptyle, N-méthylpipérazinyle, morpholino et benzène, naphthalène, anthracène, pyrène, biphényle, fluorène, spiro-9,9'-bifluorène, phénanthrène, triptycène, pyridine, furane, thiophène, pyrrole, quinoline, quinoxaline, pyrimidine et pyrazine substitués ou non substitués.

4. Procédé selon une ou plusieurs des revendications 1 à 3, **caractérisé en ce que** les bases utilisées sont des bases inorganiques, des bases organiques et également des mélanges afférents.

5. Procédé selon la revendication 4, **caractérisé en ce que** les bases inorganiques utilisées sont des carboxylates, des carbonates, des carbonates d'hydrogène et des phosphates alcalins et alcalino-terreux, et les bases organiques utilisées sont des alkoxydes métalliques du type MO-R3, où M est un métal électropositif.

6. Procédé selon la revendication 5, **caractérisé en ce que** les bases inorganiques utilisées sont acétate, carbonate et phosphate de sodium et acétate, carbonate et phosphate de potassium.

7. Procédé selon la revendication 5, **caractérisé en ce que** le métal électropositif M est lithium, sodium, potassium, magnésium ou zinc.

8. Procédé selon une ou plusieurs des revendications 1 à 5 ou 7, **caractérisé en ce que** le radical R3 représente méthyle, éthyle, propyle, *iso*-propyle, butyle, *iso-*butyle, *sec*-butyle, *tert*-butyle, *tert-*pentyle, *tert*-hexyle ou phényle.

9. Procédé selon une ou plusieurs des revendications 1 à 5 ou 8, **caractérisé en ce que** la base organique utilisée est un alkoxyde métallique du type MO-R3, où R3 représente méthyle, éthyle, propyle, *iso*-propyle, butyle, *iso*-butyle, *sec*-butyle, *tert*-butyle, *tert*-pentyle, *tert*-hexyle ou phényle.

10. Procédé selon une ou plusieurs des revendications 1 à 9, **caractérisé en ce que** les radicaux alkyle, aryle ou hétéroaryle R4 et R5 représentent méthyle, éthyle, propyle, *iso*-propyle, butyle, *iso*-butyle, sec-butyle, *tert*-butyle, *tert*-pentyle, *tert*-hexyle, cyclopentyle, cyclohexyle et phényle, o-tolyle, 2,6-diméthylphényle, mésityle, 2-*iso-*propylphényle, 2,6-di-*iso*-propylphényle, 2-*tert*-butylphényle, 2-méthoxyphényle, 2,6-diméthoxyphényle, 2,4,6-triméthoxyphényle, 2-biphényle.

11. Procédé selon la revendication 1 et/ou 10, **caractérisé en ce que** les radicaux R4 et R5 correspondent au radical *tert*-butyle.

12. Procédé selon une ou plusieurs des revendications 1 à 11, **caractérisé en ce que**, en tant que composé de nickel, on utilise des composés de nickel(II), de préférence des halogénures de nickel(II), des biscarboxylates de nickel(II), des cétonates de nickel(II), ou des complexes simples qui peuvent en être dérivés, tels que des halogénures nickel(II) oléfine, des halogénures de nickel(II) allyle ou également un nickel métallique dispersé ou colloïdal, supporté ou non supporté, ou également d'autres composés de nickel(0).

13. Procédé selon la revendication 12, **caractérisé en ce que** les composés de nickel(II) utilisés sont des halogénures de nickel(II), des biscarboxylates de nickel(II) ou des cétonates de nickel(II), et les complexes dérivables utilisés sont des halogénures de nickel(II) oléfine ou des halogénures de nickel(II) allyle.

14. Procédé selon une ou plusieurs des revendications 1 à 11, **caractérisé en ce que**, en tant que composé de palladium, on utilise des composés de palladium(II) ou des complexes simples qui peuvent en être dérivés ou un palladium métallique dispersé ou colloïdal, supporté ou non supporté, ou également d'autres composés de palladium(0).

15. Procédé selon la revendication 14, **caractérisé en ce que** les composés de palladium(II) utilisés sont des halogénures de palladium(II), des biscarboxylates de palladium(II), des cétonates de palladium(II), ou les complexes dérivables utilisés sont des halogénures de palladium(II) nitrile, des halogénures de palladium(II) oléfine ou des halogénures de palladium(II) allyle.

16. Procédé selon une ou plusieurs des revendications 1 à 11, 14 et 15, **caractérisé en ce que** le composé de palladium utilisé est acétate de palladium(II) ou Pd₂(dba)₃.

17. Procédé selon une ou plusieurs des revendications 1 à 16, **caractérisé en ce que**, en tant que solvant inerte, on utilise des solvants aromatiques, des éthers, des éthers et des polyéthers cycliques, de l'éther diméthylique ou éthylique de di-, tri- et tétraéthylène glycol ou de l'éther diméthylique ou éthylique d'oligo- ou polyéthylène glycol, des N,N-dialkylcarboxamides et des N-alkyllactones, en option également des mélanges de ces solvants.

18. Procédé selon la revendication 17, **caractérisé en ce que** le solvant aromatique utilisé est du toluène et/ou les xylènes isomériques, du diméthylformamide, du diméthylacétamide ou du N-méthylpyrrolidin-2-one, en option également des mélanges de ces solvants.

19. Procédé selon la revendication 1, **caractérisé en ce que** l'utilisation de diamines ou de dihaloarylènes en tant que matériaux de départ permet la synthèse de composés oligomérique ou polymériques.
